# FASCICULE DE BREVET EUROPEEN

(11) **EP 3 830 085 B1**
(45) Date de publication et mention de la délivrance du brevet: **08.11.2023**
(21) Numéro de dépôt: 19759658.8
(22) Date de dépôt: 26.07.2019
(51) Int. Cl.: A61K 31/428, A61P 1/16, C07D 419/12, A61P 1/00, A61P 11/00, A61P 37/00, A61P 13/12, C07D 417/12

(54) **DERIVES DEUTERES DU LANIFIBRANOR**
DEUTERIERTE DERIVATE VON LANIFIBRANOR
DEUTERATED DERIVATIVES OF LANIFIBRANOR

(30) Priorité: 27.07.2018 FR 1857021
(43) Date de publication de la demande: 09.06.2021
(60) Demande divisionnaire: 23201458.9
(73) Titulaire: Inventiva, 21121 Daix (FR)
(72) Inventeur: MONTALBETTI, Christian, 21121 FONTAINE-LES-DIJON (FR); BOUBIA, Benaissa, 21850 SAINT APOLLINAIRE (FR)
(74) Mandataire: Cabinet Beau de Loménie
(86) Numéro de dépôt international: PCT/FR2019/051860
(87) Numéro de publication internationale: WO 2020/021215

(56) Documents cités:
- BOUBIA ET AL.: "DESIGN, SYNTHESIS AND EVALUATION OF A NOVEL SERIES OF INDOLE SULFONAMIDE PEROXISOME PROLIFERATOR ACTIVATED RECEPTOR (PPAR) ALPHA/GAMMA/DELTA TRIPLE ACTIVATORS: DISCOVERY OF LANIFIBRANOR A NEW ANTIFIBROTIC CLINICAL CANDIDATE", MEDICINAL CHEMISTRY, vol. 61, 15 février 2018 (2018-02-15), pages 2246-2265, XP002790345,
- KATSNELSON A.: "HEAVY DRUGS DRAW HEAVY INTEREST FROM PHARMA BACKERS", NATURE MEDICINE, vol. 19, no. 6, 1 juin 2013 (2013-06-01), page 656, XP002790346, cité dans la demande

## Description

### Domaine de l'invention

La présente invention concerne des dérivés deutérés du lanifibranor, notamment pour leur usage en thérapie, en particulier dans le traitement des maladies fibrotiques.

### Etat de la technique

Le lanifibranor ou acide 4-(1-(1,3-Benzothiazol-6-ylsulfonyl)-5-chloro-indol-2-yl)-butanoïque est un agoniste des récepteurs PPAR utile notamment pour son usage dans le traitement de la sclérodermie systémique (SSc) et dans la stéatose hépatique non alcoolique (NASH). Le lanifibranor est notamment décrit avec d'autres dérivés indoles dans la demande de brevet WO 2007/026097, notamment pour la prévention ou le traitement des hypertriglycéridémies, des hypercholestérolémies et, d'une façon plus générale, le rétablissement de paramètres normaux lors d'une perturbation du métabolisme lipidique et glucidique, et également dans le cas du traitement de la dysfonction endothéliale, de maladies inflammatoires ou de neurodégénérescences.

On connaît l'intérêt d'employer des molécules deutérées comme moyen de modifier leur métabolisme en améliorant leur stabilité métabolique et éventuellement leurs propriétés de pénétration cellulaire (Maehr & al., J. Med. Chem. 2013, 56, 3878-3888 ; Kerekes & al., J. Med. Chem. 2011, 54, 201-210 ; Harbeson & Tung, Medchem News 2014, 2 9-22 ; Gant, J. Med. Chem. 2014, 57, 3595-3611 ; DeWitt & Maryanoff, Biochemistry 2018, 57, 472-473 ; WO 2017/136375 ; WO 2018/039521). La synthèse de médicaments deutérés est également décrite dans plusieurs articles (Modutwa & a., J. Label Compd. Radiopharm 2010, 53 686-692 ; Junk & al., J. Label Compd. Radiopharm 1997, 39 625-630).

Toutefois, il est reconnu que l'effet des substitutions d'hydrogènes par des deutériums sur le métabolisme des produits chimiques reste imprévisible et pour de nombreux auteurs, la deutération des médicaments n'est pas considérée comme un moyen de modulation fiable (Foster, AB, Adv Drug Res 1985, 14: 1-40; Fisher, MB et al, Curr Opin Drug Discov Devel, 2006, 9: 101-09 ; Katsnelson, Nature Medecine 2013, 19 6 656).

### Exposé de l'invention

L'invention concerne un dérivé deutéré du lanifibranor de formule (I) : 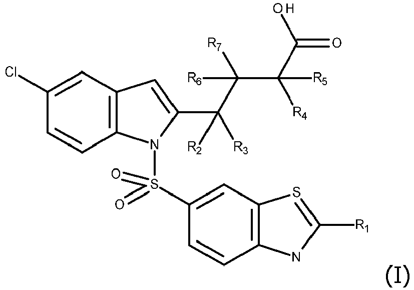 dans laquelle au moins l'un des groupes R₁ à R₇ est un atome de deutérium (D) et les autres groupes R₁ à R₇ sont des atomes d'hydrogène (H).

L'invention concerne également une composition, en particulier une composition pharmaceutique, qui comprend au moins un dérivé deutéré de formule (I).

L'invention concerne aussi les dérivés deutérés de formule (I) pour leur utilisation en thérapie, en particulier pour le traitement de maladies fibrotiques.

### Description détaillée de l'invention

L'invention concerne un dérivé deutéré du lanifibranor de formule (I) : 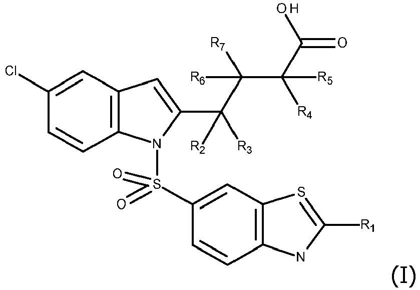 dans laquelle au moins l'un des groupes R₁ à R₇ est un atome de deutérium et les autres groupes R₁ à R₇ sont des atomes d'hydrogène, et ses sels et solvates pharmaceutiquement acceptables.

Selon un premier mode de réalisation de l'invention, au moins le groupe R₁ est D.

En particulier le dérivé deutéré du lanifibranor est l'acide 4-(1-(2-deutério-1,3-benzothiazol-6-yl)sulfonyl)-5-chloro-1H-indol-2-yl)butanoïque.

Selon un autre mode de réalisation de l'invention, au moins l'un des groupes R₂ à R₇ est D. Plus préférentiellement au moins l'un des groupes R₂ et R₃ et/ou au moins l'un des groupes R₄ et R₅ et/ou au moins l'un des groupes R₆ et R₇ est D. Encore plus préférentiellement, R₂, R₃, R₄, R₅, R₆ et R₇ sont D.

En particulier, le dérivé deutéré du lanifibranor est l'acide 4-[1-(1,3-benzothiazol-6-ylsulfonyl)-5-chloro-indol-2-yl]-2,2,3,3,4,4-hexadeutériobutanoïque.

Les sels pharmaceutiquement acceptables des dérivés deutérés selon l'invention sont les mêmes que les sels pharmaceutiquement acceptables du lanifibranor, notamment par combinaison de l'acide avec une base minérale ou organique non toxique pharmaceutiquement acceptable. Parmi les bases minérales, on peut utiliser par exemple les hydroxydes de sodium, de potassium, de magnésium ou de calcium. Parmi les bases organiques, on peut utiliser par exemple les amines, les aminoalcools, des acides aminés basiques tels que la lysine ou l'arginine ou encore des composés porteurs d'une fonction ammonium quaternaire tels que par exemple la bétaïne ou la choline.

Les composés selon l'invention sont préparés selon les méthodes usuelles de préparation du lanifibranor, par exemple celle décrite dans la demande de brevet WO 2007/026097, les intermédiaires de synthèse deutérés venant remplacer les mêmes intermédiaires non enrichis isotopiquement employés dans ces méthodes usuelles.

L'invention concerne également une composition comprenant un dérivé deutéré selon l'invention, ou l'un de ses sels ou solvates, notamment un sel ou solvate pharmaceutiquement acceptable, et un support approprié à son usage.

Pour un usage en thérapie, la composition selon l'invention est avantageusement une composition pharmaceutique, le support comprenant des excipients usuels de la pharmacopée, choisis selon le mode d'administration envisagé.

De telles compositions sont connues de l'homme du métier, et notamment décrites dans les demandes de brevet WO 2007/026097 et WO 2015/189401.

Pour une administration par voie orale, par exemple sous la forme de comprimé, gélule, pastille, gel, sirop, suspension buvable, la composition pharmaceutique selon l'invention comprend avantageusement de 1 à 1000 mg de dérivé deutéré de formule (I), par exemple 1 mg, 5 mg, 10 mg, 50 mg, 100 mg, 200 mg, 500 mg ou 1000 mg.

L'invention concerne aussi les dérivés deutérés de formule (I), ou leurs sels ou solvates pharmaceutiquement acceptables, pour leur utilisation en thérapie.

Les différents usages thérapeutiques des dérivés deutérés selon l'invention sont ceux connus du lanifibranor et ses analogues, tels que ceux décrits dans les demandes de brevet WO 2007/026097 et WO 2015/189401.

L'invention concerne donc les dérivés deutérés de formule (I), ou leurs sels ou solvates pharmaceutiquement acceptables, pour leur utilisation pour lutter contre les hypercholestérolémies, les hyperlipidémies, les hypertriglycéridémies, les dyslipidémies, l'insulinorésistance, le diabète ou l'obésité ainsi que les maladies cardiovasculaires qui sont la conséquence d'un déséquilibre des lipoprotéines sériques. Les composés selon l'invention sont également utiles comme principes actifs de médicaments destinés à prévenir ou traiter les maladies liées à un dysfonctionnement endothélial, l'athérosclérose, l'infarctus du myocarde, l'hypertension, les problèmes cérébrovasculaires, certaines maladies inflammatoires comme par exemple l'arthrite rhumatoïde, et les neurodégénérescences comme notamment la maladie d'Alzheimer ou la maladie de Parkinson.

L'invention concerne en particulier les dérivés deutérés de formule (I) pour leur utilisation dans le traitement des maladies fibrotiques. Dans un mode de réalisation, la maladie fibrotique est une condition affectant tout organe qui peut développer une fibrose, tel que le coeur, le poumon, le foie, le rein, le tractus gastro-intestinal, la peau, les muscles, etc. La maladie fibrotique est en particulier choisie parmi: la fibrose hépatique, la stéatose hépatique, la stéatohépatite non alcoolique, la maladie rénale chronique, un trouble fibrotique pulmonaire comme la fibrose pulmonaire idiopathique et la sclérodermie systémique.

Le besoin de traitement du patient aura avantageusement été déterminé auparavant par toute méthode de diagnostic appropriée à la détection de la maladie fibrotique à traiter, notamment par l'analyse des niveaux d'expression des récepteurs PPAR dans les tissus fibrotiques des patients à traiter.

### Exemples

### Abréviations

APCI = ionisation chimique à pression atmosphérique
DCM = dichlorométhane
DMSO = diméthylsulfoxyde
éq. = équivalent
EtOAc = acétate d'éthyle
EtOH = éthanol
h = heure
HPLC = chromatographie en liquide à haute performance
LCMS = chromatographie en phase liquide couplée à la spectrométrie de masse
MeOH = méthanol
min = minute
Pd(PPh₃)₄ = tétrakis(triphénylphosphine)palladium(0)
pTsOH = acide paratoluènesulfonique
RMN = résonance magnétique nucléaire
THF = tétrahydrofurane

### Exemple 1 : acide 4-[5-Chloro-1-[(2-deutério-1,3-benzothiazol-6-yl)sulfonyl)]indol-2-yl]butanoïque

### Préparation 1 : 2-Deutério-1,3-benzothiazole

A partir du 1,3-benzothiazole (5,0 g, 36,9 mmol, 1,0 éq.) dissous dans le THF (80 mL), et refroidi à -78 °C du *n*-BuLi (37,0 mL, 92,5 mmol, 2.5 éq.; solution 2,5 M dans l'hexane) a été additionné goutte à goutte. Après addition complète, D₂O (4 mL, 222 mmol, 6,0 éq.) a été ajouté au mélange réactionnel à -78 °C, le tout a été agité pendant 30 min puis amené à température ambiante. Le suivi de la réaction a été assuré par analyse LCMS. Le mélange réactionnel a alors été traité par une solution de NH₄Cl aqueuse saturée et le tout a été extrait 3 fois au DCM. Les phases organiques ont été regroupées et séchées sur MgSO₄ puis filtrées et évaporées sous pression réduite pour conduire à un brut purifié par chromatographie flash (éluant: 0 à 40% EtOAc dans du *n*-heptane) pour délivrer le composé de la **préparation 1** (3,22 g, rendement 64%) sous forme d'huile brune. APCI MS m/z 137 [M+H]⁺; HPLC-MS (220-254 nm) pureté : 99%. ¹H RMN (300 MHz DMSO-*d₆*): δ 7.46-7.58 (2H, m); 8.08-8.19 (2H, m).

### Préparation 2 : 2-Deutério-6-nitro-1,3-benzothiazole

Le 2-deutério-1,3-benzothiazole (**préparation 1,** 2,9 g, 21,3 mmol, 1,0 éq.) a été additionné lentement sur une solution d'acide sulfurique (13 mL) à 0°C, de l'acide nitrique (6,4 mL) a ensuite été additionné goutte à goutte tout en maintenant la température en dessous de 0°C. Le mélange réactionnel a alors été amené à température ambiante et agité pendant 12 h avant d'être hydrolysé sur un mélange d'eau glacée. Le précipité jaune a été filtré, lavé à l'eau et cristallisé dans l'éthanol pour conduire au produit désiré (**préparation 2,** 1,4 g, rendement 37%) sous forme de cristaux jaunes. APCI MS m/z 182 [M+H]⁺; HPLC-MS (220-254 nm) pureté >99%. ¹H RMN (300 MHz DMSO-d6): δ 8.27-8.38 (2H, m); 9.25 (1H, d, J=2.3 Hz).

### Préparation 3 : 2-Deutério-1,3-benzothiazol-6-amine

A partir du composé de la **préparation 2** (2,0 g, 11,0 mmol, 1,0 éq.) dissous dans un mélange 1:1 EtOH - EtOAc (60 mL), de la poudre de chlorure d'étain (7,3 g, 38,6 mmol, 3,5 éq.) a été ajoutée et la solution a été agitée à température ambiante durant 4 h. A la fin de la réaction le mélange réactionnel a été filtré à travers une cartouche de Celite^{™} et extrait par une solution saturée de Na₂CO₃. Les phases organiques ont été regroupées et séchées sur MgSO₄ puis filtrées et évaporées sous pression réduite pour conduire à un brut purifié par chromatographie flash (éluant: 0 à 20% MeOH dans DCM) pour obtenir le composé désiré (**préparation 3,** 970 mg, rendement 58%) sous forme de cristaux marron. APCI MS m/z 152 [M+H]⁺; HPLC-MS (220-254 nm) pureté : 97%. ¹H RMN (300 MHz DMSO-d6): δ 5.38 (2H, s); 6.8 (1H, dd, J=8.7 Hz et J=2.2 Hz); 7.11 (1H, d, J=2.2 Hz); 7.7 (1H, d, J=8.7 Hz).

### Préparation 4 : chlorure de 2-Deutério-1,3-benzothiazole-6-sulfonyle

Une solution aqueuse (4,5mL) de chlorure de thionyle (1,1 mL) a été préparée à 0°C et stockée une nuit à 4°C. Dans cette solution du chlorure de cuivre (I) (0,05 éq.) a été additionné à -10 °C. Le composé de la **préparation 3** (450 mg, 2,9 mmol, 1,0 éq.) a été dissous dans de l'acide chlorhydrique (3,5 mL) en maintenant la température en dessous de 25°C, cette dernière solution ayant alors été refroidie à -10°C et une solution de nitrite de sodium (3,3 mmol, 0,7 mL, 1.1 éq.) a été additionnée sans que la température dépasse -2°C. Le mélange ainsi obtenu a été agité pendant 15 min à -2°C puis additionné goutte à goutte à la première solution de chlorure de thionyle à -5°C. Le mélange réactionnel a alors été agité à -5°C pendant 3 h puis la réaction a été hydrolysée dans de l'eau. Le précipité formé a été filtré puis lavé à l'eau pour conduire au produit désiré (**préparation 4,** 255 mg, rendement 37%) sous forme de cristaux marron clair. Ce composé a été utilisé dans l'étape suivante sans aucune purification. HPLC-MS (220-254 nm) pureté : 94%.

### Préparation 5: N-(4-Chloro-2-iodophényl)-2-deutério-1,3-benzothiazole-6-sulfonamide

Sous argon, de la 4-chloro-2-iodoaniline (564 mg, 2,2 mmol, 1,0 éq.) a été dissoute dans de la pyridine anhydre (8 mL) et le composé de la **préparation 4** (600 mg, 2,5 mmol, 1,15 éq.) a été additionné. La solution a alors été agitée à température ambiante pendant 2 h. Le solvant a été évaporé et le brut réactionnel a été purifié par chromatographie flash (éluant: 0 à 30% EtOAc dans du n-heptane;) pour conduire au produit désiré **(préparation 5,** 0,73 g, rendement 73%) sous forme de cristaux marron. APCI MS m/z 452 [M+H]⁺; HPLC-MS (220-254 nm) pureté : 94%. ¹H RMN (300 MHz DMSO-d6): δ 7.01 (1H, d, J=8.5 Hz); 7.4 (1H, dd, J=8.5 Hz et J=2.3 Hz); 7.82 (1H, dd, J=8.7 Hz et J=1.8 Hz); 7.88 (1H, d, J=2.4 Hz); 8.26 (1H, d, J=8.7 Hz); 8.6 (1H, d, J=1.5 Hz); 10.03 (1H, s).

### Préparation 6: acide 4-[5-Chloro-1-[(2-deutério-1,3-benzothiazol-6-yl)sulfonyl)]indol-2-yl]butanoïque

Sous argon le composé de la **préparation 5** (1,31 g, 2,9 mmol, 1,0 éq.) a été dissout dans du DMF anhydre (30 mL), ensuite de l'acide 5-hexynoïque (360 mg, 3,2 mmol, 1,1 éq.), de l'iodure de cuivre (56 mg, 0,3 mmol, 0,1 éq.), du Pd(PPh₃)₄ (168 mg, 0,15 mmol, 0,05 éq.) et de la triéthylamine (606 µL, 4,4 mmol, 1,5 éq.) ont été additionnés et le mélange réactionnel a été agité à 80°C pendant 2 h. A la fin de la réaction le mélange a été repris avec une solution aqueuse 1N d'acide chlorhydrique et extrait deux fois par de l'acétate d'éthyle. Les phases organiques ont été regroupées et séchées sur MgSO₄ puis filtrées et évaporées sous pression réduite pour conduire à un brut purifié par chromatographie flash (éluant: 0 à 5% MeOH dans DCM) pour conduire au produit désiré, (**Exemple 1,** 1,05 g, rendement 83%) sous forme de cristaux marron. APCI MS m/z 452 [M+H]⁺; HPLC-MS (220-254 nm) pureté : 94%. ¹H RMN (300 MHz DMSO-d6): δ 1.86-2.01 (2H, m); 2.29-2.41 (2H, m); 3.08 (2H, t, J=7.2 Hz); 6.61 (1H, s); 7.31 (1H, dd, J=8.7 Hz et J=2.0 Hz); 7.51 (1H, d, J=2.0 Hz); 7.84 (1H, dd, J=8.7 Hz et J=1.8 Hz); 8.08 (1H, d, J=8.7 Hz); 8.19 (1H, d, J=8.7 Hz); 8.97 (1H, d, J=1.8 Hz); 12.13 (1H, s). Taux d'incorporation du deutérium dosé par RMN 500MHz : 98%.

### Exemple 2: Acide 4-[1-(1,3-benzothiazol-6-ylsulfonyl)-5-chloro-indol-2-yl]-2,2,3,3,4,4-hexadeutério-butanoïque.

### Préparation 7: 2-(1,1,2,2,3,3,4,4-octadeutério-4-iodo-butoxy)-tétra-hydropyrane

De l'iodure de triméthylsilyle (25 g, 125 mmol, 1 éq.) a été additionné à 0°C à du THF-D8 (10 g, 125 mmol, 1 éq.). Après agitation pendant 2 h à 0°C, de l'éther (80 mL) et de l'eau (20 mL) ont été additionnés. Le mélange a été agité pendant 3 h puis décanté. La phase organique a alors été séchée sur MgSO₄, filtrée et concentrée sous pression réduite. Le brut réactionnel ainsi obtenu a été repris dans du DCM (100 mL) à 0°C, puis du dihydropyrane (12,5 mL, 150 mmol, 1,2 éq.) et du pTsOH (50 mg, 0,2 mmol, 0,002 éq.) ont été additionnés. Le mélange réactionnel a ensuite été agité à 0°C pendant une nuit. La solution a été lavée successivement à l'aide d'une solution saturée de NaHCO₃ (3 x 15 mL), puis de saumure puis séchée sur du Na₂SO₄ avant d'être concentrée sous pression réduite. Le brut réactionnel a été purifié par chromatographie flash (éluant: 1:20 jusqu'à 1:5 EtOAc:Hexane) pour conduire au produit désiré (**Préparation 7,** 23 g, rendement 62%) sous forme d'une huile incolore qui a été utilisée directement dans l'étape suivante.

### Préparation 8 : 2-(1,1,2,2,3,3,4,4-octadeutériohex-5-ynoxy)tétrahydropyrane

Dans une solution d'acétylure de lithium (19,94 g, 186,3 mmol, 1 éq.) dans du DMSO (60mL) fraîchement distillé a été additionnée goutte à goutte à 5°C une solution du composé de la **préparation 7** (30 g, 102,7 mmol, 0,55 éq.) dans du DMSO (30 mL). Cette solution ainsi obtenue a été agitée pendant 2 h puis hydrolysée par une solution de NH₄Cl à 0°C. Ce mélange a alors été extrait par de l'hexane et lavé à l'aide d'une solution de sulfate de cuivre (5%, 20 mL) puis à l'aide de saumure (20 mL). Les phases organiques ont été regroupées et séchées sur Na₂SO₄ avant d'être concentrées sous pression réduite, pour conduire au produit désiré (**Préparation 8,** 10,7 g, rendement 55%) sous forme d'une huile jaune qui a été utilisée directement dans l'étape suivante sans aucune purification.

### Préparation 9 : 1,1,2,2,3,3,4,4-octadeutériohex-5-yn-1-ol

A une solution du composé de la **préparation 8** (10,7 g, 56,2 mmol, 1 éq.) dans un mélange de THF (12,5 mL) et de MeOH (350 mL) à 0°C, a été additionné par petites portions du pTsOH (360 mg, 1,82 mmol, 0,03 éq.) et le mélange réactionnel ainsi obtenu a été agité pendant une nuit. Le mélange a ensuite été lavé à l'aide d'une solution saturée de NaHCO₃ (3 x 15 mL) et de saumure (15 mL), puis séché sur Na₂SO₄ avant d'être concentré sous pression réduite. Le brut réactionnel a été purifié par chromatographie flash (éluant: 1:20 jusqu'à 1:5 EtOAc:Hexane) pour conduire au produit désiré **(Préparation 9,** 5 g, rendement 82%) sous forme d'une huile incolore qui a été utilisée directement dans l'étape suivante.

### Préparation 10 : acide 2,2,3,3,4,4-hexadeutériohex-5-ynoïque

A une solution du composé de la **préparation 9** (9,25 g, 87 mmol, 1 éq.) dans de l'acétone (87 mL) et sous agitation a été ajouté du réactif de Jones (CrO₃ (17,45 g, 174,5 mmol, 2 éq.) dans H₂SO₄ 10N (218 mL)) en 5 min à 0°C. Le mélange réactionnel a alors été agité pendant 1 h avant d'être concentré sous pression réduite. Puis de l'éther (130 mL) et de l'eau (4 mL) ont été additionnés immédiatement. Le solide obtenu a été filtré et le filtrat a été extrait par de l'éther (6 x 100 mL). Les phases organiques ont été regroupées et lavées avec de l'eau puis séchées sur Na₂SO₄ avant d'être filtrées et concentrées sous pression réduite pour conduire au produit désiré (**Préparation 10,** 9,17 g, rendement 89%) sous forme d'une huile orange qui a été utilisée directement dans l'étape suivante sans aucune purification.

### Préparation 11: acide 4-[1-(1,3-benzothiazol-6-ylsulfonyl)-5-chloro-indol-2-yl]-2,2,3,3,4,4-hexadeutériobutanoïque

Un mélange d'acide benzothiazole-6-sulfonique (4-chloro-2-iodo-phényl)-amide (16 g, 35,5 mmol, 1 éq.), d'acide hex-5-ynoïque (**préparation 10,** 5,2 g, 44 mmol, 1,24 éq.), d'iodure de cuivre (335 mg, 1,75 mmol, 0,05 éq.), de dichlorobis(triphényl-phosphine)palladium (1,24 g, 1,72 mmol, 0.05 éq.), de triéthylamine (130 mL) et de N,N-diméthylformamide (130 mL) a été agité sous azote à 110°C pendant 1 h. Le mélange réactionnel a alors été dilué avec une solution 1M de HCl et extrait à l'acétate d'éthyle, les insolubles ont été filtrés et le filtrat a été séché sur Na₂SO₄ avant d'être concentré sous pression réduite. Le brut réactionnel a été purifié par chromatographie flash puis recristallisé dans le DCM pour conduire au produit désiré (**Exemple 2,** 2,3 g, rendement 15%) sous forme d'un solide blanc. Taux d'incorporation du deutérium dosé par RMN 500MHz : >99%. ¹H RMN (400 MHz, DMSO-d6) δ 6.62 (1H, d, J = 0.6 Hz), 7.32 (1H, dd, J = 8.9 et 2.2 Hz), 7.57 (1H, d, J = 2.1 Hz), 7.85 (1H, dd, J = 8.7 et 2.1 Hz), 8.10 (1H, d, J = 8.9 Hz), 8.20 (1H, d, J = 8.7 Hz), 8.99 (1H, s), 9.66 (1H, s), 12.15 (1H, s).

### Exemple 3 : Activité Pharmacologique

Les composés de l'invention ont été soumis à des tests biologiques de façon à évaluer leur potentiel à traiter ou prévenir certaines pathologies. Dans un premier temps, on a mesuré l'aptitude des composés à se comporter en activateur des récepteurs nucléaires PPAR.

Un test de transactivation a été utilisé comme test de screening primaire. Des cellules Cos-7 ont été transfectées avec un plasmide exprimant une chimère d'un récepteur murin ou humain PPAR-Gal4 (récepteur PPARa-Gal4 ou PPARδ-Gal4 ou PPARγ-Gal4) et d'un plasmide rapporteur 5Gal4pGL3 TK Luc. Les transfections ont été réalisées à l'aide d'un agent chimique (Jet PEI).

Les cellules transfectées ont été distribuées dans des plaques 384 puits et laissées au repos pendant 24 heures.

Au temps 24 heures le milieu de culture a été changé. Les produits à tester ont été ajoutés (concentration finale comprise entre 10⁻⁴ et 3.10⁻¹⁰ M) dans le milieu de culture. Après une nuit d'incubation, l'expression de luciférase a été mesurée après addition de « SteadyGlo » selon les instructions du fabricant (Promega).

L'acide fénofibrique à 10⁻⁵ M (agoniste PPARα), le GW501516 à 10⁻⁸ M (agoniste PPARδ) et la rosiglitazone à 10⁻⁶ M (agoniste PPARγ) ont été utilisés comme références.

Les résultats sont exprimés en taux d'induction (nombre de fois) comparativement au niveau basal en pourcentage d'activité de la référence adéquate (référence = 100 %). Les courbes effet-concentration et les EC₅₀ ont été calculées à l'aide du logiciel Assay Explorer (MDL).

**Tableau 1**

| Récepteur humain | PPARα | | PPARδ | | PPARγ | |
|---|---|---|---|---|---|---|
| Exemple | EC₅₀(nM) | Top(%) | EC₅₀(nM) | Top(%) | EC₅₀(nM) | Top(%) |
| 1 | 2317 | 67 | 952 | 80 | 85 | 53 |
| 2 | 2280 | 62 | 1177 | 80 | 136 | 50 |

Comme on peut le constater à la lecture du tableau ci-dessus, les dérivés deutérés du lanifibranor activent les trois sous-types de récepteurs PPAR (PPARα, PPARγ et PPARδ), avec une EC₅₀ inférieure à 2,5 µM pour chacun des sous-types. On peut également noter que le rapport entre les EC₅₀ de deux sous-types PPAR est soit inférieur à 100, soit supérieur à 0,01.

### Références

- 1.: DeWitt & Maryanoff, Biochemistry 2018, 57, 472-473
- 2.: Fisher et al, Curr Opin Drug Discov Devel, 2006, 9: 101-09
- 3.: Foster, Adv Drug Res 1985, 14: 1-40
- 4.: Gant, J. Med. Chem. 2014, 57, 3595-3611
- 5.: Harbeson & Tung, Medchem News 2014, 2 9-22
- 6.: Junk & al., J. Label Compd. Radiopharm 1997, 39 625-630
- 7.: Katsnelson, Nature Medecine 2013, 19 6 656
- 8.: Kerekes & al., J. Med. Chem. 2011, 54, 201-210
- 9.: Maehr & al., J. Med. Chem. 2013, 56, 3878-3888
- 10.: Modutwa & a., J. Label Compd. Radiopharm 2010, 53 686-692
- 11.: WO 2007/026097
- 12.: WO 2015/189401
- 13.: WO 2017/136375
- 14.: WO 2018/039521

## Revendications

1. Dérivé deutéré du lanifibranor de formule (I) : 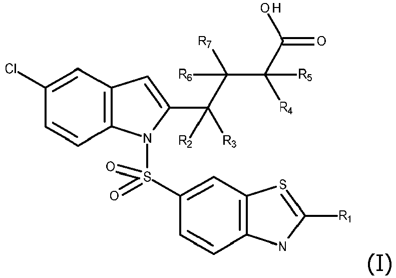 dans laquelle au moins l'un des groupes R₁ à R₇ est un atome de deutérium et les autres groupes R₁ à R₇ sont des atome d'hydrogène, et ses sels et solvates pharmaceutiquement acceptables.

2. Dérivé deutéré selon la revendication 1, dans lequel au moins le groupe R₁ est D.

3. Dérivé deutéré selon la revendication 2, qui est l'acide 4-(1-(2-deutério-1,3-benzothiazol-6-yl)sulfonyl)-5-chloro-1H-indol-2-yl)butanoïque.

4. Dérivé deutéré selon la revendication 1, dans lequel au moins l'un des groupes R₂ à R₇ est D.

5. Dérivé deutéré selon la revendication 4, dans lequel au moins l'un des groupes R₂ et R₃ et/ou au moins l'un des groupes R₄ et R₅ et/ou au moins l'un des groupes R₆ et R₇ est D.

6. Dérivé deutéré selon la revendication 5, qui est l'acide 4-[1-(1,3-benzothiazol-6-ylsulfonyl)-5-chloro-indol-2-yl]-2,2,3,3,4,4-hexadeutériobutanoïque.

7. Composition qui comprend un dérivé deutéré selon l'une des revendications 1 à 6, ou l'un de ses sels ou solvates, et un support approprié.

8. Composition pharmaceutique qui comprend un dérivé deutéré selon l'une des revendications 1 à 6, ou un sel ou solvate pharmaceutiquement acceptable de celui-ci, et un support pharmaceutiquement acceptable.

9. Dérivé deutéré selon l'une des revendications 1 à 6, ou un sel ou solvate pharmaceutiquement acceptable de celui-ci, pour son utilisation en thérapie.

10. Dérivé deutéré selon l'une des revendications 1 à 6, ou un sel ou solvate pharmaceutiquement acceptable de celui-ci, pour son utilisation dans le traitement des maladies fibrotiques, en particulier choisies parmi: la fibrose hépatique, la stéatose hépatique, la stéatohépatite non alcoolique, la maladie rénale chronique, un trouble fibrotique pulmonaire comme la fibrose pulmonaire idiopathique, et la sclérodermie systémique.

## Patentansprüche

1. Ein deuteriertes Derivat von Lanifibranor der Formel (I): 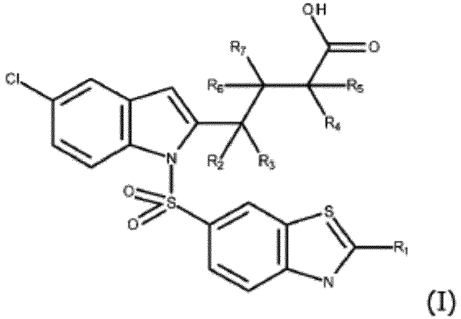 worin mindestens eine der Gruppen R₁ bis R₇ ein Deuteriumatom ist und die anderen Gruppen R₁ bis R₇ Wasserstoffatome sind, und seine pharmazeutisch akzeptablen Salze und Solvate.

2. Deuteriertes Derivat nach Anspruch 1, wobei mindestens die Gruppe R₁ D ist.

3. Deuteriertes Derivat nach Anspruch 2, das 4-(1-(2-Deuterio-1,3-benzothiazol-6-yl)sulfonyl)-5-chloro-1H-indol-2-yl)butansäure ist.

4. Deuteriertes Derivat nach Anspruch 1, wobei mindestens eine der Gruppen R₂ bis R₇ D ist.

5. Deuteriertes Derivat nach Anspruch 4, wobei mindestens eine der Gruppen R₂ und R₃ und/oder mindestens eine der Gruppen R₄ und R₅ und/oder mindestens eine der Gruppen R₆ und R₇ D ist.

6. Deuteriertes Derivat nach Anspruch 5, das 4-[1-(1,3-Benzothiazol-6-ylsulfonyl)-5-chlor-indol-2-yl]-2,2,3,3,4,4-hexadeuteriobutansäure ist.

7. Zusammensetzung, umfassend ein deuteriertes Derivat nach einem der Ansprüche 1 bis 6 oder eines seiner Salze oder Solvate und einen geeigneten Träger.

8. Pharmazeutische Zusammensetzung, die ein deuteriertes Derivat nach einem der Ansprüche 1 bis 6 oder ein pharmazeutisch annehmbares Salz oder Solvat davon und einen pharmazeutisch annehmbaren Träger enthält.

9. Deuteriertes Derivat nach einem der Ansprüche 1 bis 6 oder ein pharmazeutisch annehmbares Salz oder Solvat davon zur Verwendung in der Therapie.

10. Deuteriertes Derivat nach einem der Ansprüche 1 bis 6 oder ein pharmazeutisch annehmbares Salz oder Solvat davon zur Verwendung bei der Behandlung von fibrotischen Erkrankungen, insbesondere ausgewählt aus: Leberfibrose, Lebersteatose, nichtalkoholische Steatohepatitis, chronische Nierenerkrankung, eine fibrotische Lungenerkrankung wie idiopathische Lungenfibrose und systemische Sklerose.

## Claims

1. A deuterated derivative of lanifibranor of formula (I): 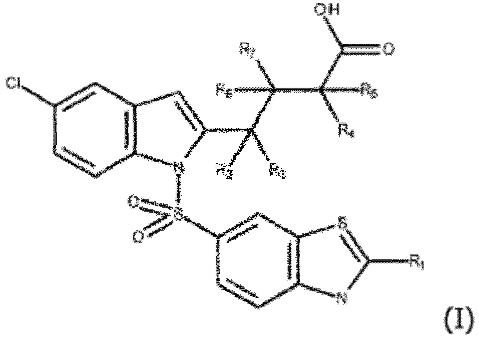 wherein at least one of the groups R₁ to R₇ is a deuterium atom and the other groups R₁ to R₇ are hydrogen atoms, and its pharmaceutically acceptable salts and solvates.

2. The deuterated derivative of claim 1, wherein at least the group R₁ is D.

3. The deuterated derivative of claim 2, which is 4-(1-(2-deuterio-1,3-benzothiazol-6-yl)sulfonyl)-5-chloro-1H-indol-2-yl)butanoic acid.

4. The deuterated derivative of claim 1, wherein at least one of the groups R₂ to R₇ is D.

5. The deuterated derivative of claim 4, wherein at least one of the groups R₂ and R₃ and/or at least one of the groups R₄ and R₅ and/or at least one of the groups R₆ and R₇ is D.

6. The deuterated derivative of claim 5, which is 4-[1-(1,3-benzothiazol-6-ylsulfonyl)-5-chloro-indol-2-yl]-2,2,3,3,4,4-hexadeuteriobutanoic acid.

7. A composition which comprises a deuterated derivative of one of claims 1 to 6, or one of its salts or solvates, and a suitable carrier.

8. A pharmaceutical composition which comprises a deuterated derivative of one of claims 1 to 6, or a pharmaceutically acceptable salt or solvate thereof, and a pharmaceutically acceptable carrier.

9. A deuterated derivative of one of claims 1 to 6, or a pharmaceutically acceptable salt or solvate thereof, for use in therapy.

10. A deuterated derivative of one of claims 1 to 6, or a pharmaceutically acceptable salt or solvate thereof, for use in the treatment of fibrotic diseases, in particular chosen from: liver fibrosis, liver steatosis, non-alcoholic steatohepatitis, chronic kidney disease, a pulmonary fibrotic disorder such as idiopathic pulmonary fibrosis, and systemic sclerosis.
